# EUROPÄISCHE PATENTSCHRIFT

(11) **EP 3 419 606 B1**
(45) Veröffentlichungstag und Bekanntmachung des Hinweises auf die Patenterteilung: **25.12.2019**
(21) Anmeldenummer: 17701268.9
(22) Anmeldetag: 24.01.2017
(51) Int. Cl.: A61K 9/16, B01J 2/22, C07C 229/08

(54) **GLYCIN-PARTIKEL, SOWIE VERFAHREN ZUR DEREN HERSTELLUNG UND DEREN VERWENDUNG**
GLYCINE PARTICLES, PREPARATION METHOD AND USE THEREOF
PARTICULES DE GLYCINE, LEUR MÉTHODE DE PRÉPARATION ET LEUR UTILISATION

(30) Priorität: 23.02.2016 EP 16000431
(43) Veröffentlichungstag der Anmeldung: 02.01.2019
(73) Patentinhaber: Merck Patent GmbH, 64293 Darmstadt (DE)
(72) Erfinder: BENK, Harald, 64331 Weiterstadt (DE); BIRK, Gudrun, 64285 Darmstadt (DE); DECKER, Melina, 64673 Zwingenberg (DE); LUBDA, Dieter, 64625 Bensheim (DE); MODDELMOG, Guenter, 64354 Reinheim (DE); MUESSIG, Harald, 63853 Moemlingen (DE); SCARAMUZZA, Tanino, 64739 Hoechst (DE); SCHLEEHAHN, Michael, 64291 Darmstadt (DE)
(86) Internationale Anmeldenummer: PCT/EP2017/000078
(87) Internationale Veröffentlichungsnummer: WO 2017/144149

(56) Entgegenhaltungen:
- DE-A1- 2 622 462
- DE-T2- 69 706 988

## Beschreibung

Die vorliegende Erfindung betrifft kompaktiertes Glycin - Granulat dessen Korngröße über 0,7 mm liegt, sowie Verfahren zur Herstellung und Verwendung derartiger Granulate.

Glycin (Aminoessigsäure) ist eine α-Aminosäure, welche nicht chiral und damit auch nicht optisch aktiv ist. Gylcin fällt typischerweise als ein farbloser, kristalliner Feststoff an, welcher gut wasserlöslich ist. Der Schmelzpunkt liegt bei etwa 234°C (Zersetzung).
Glycin erfüllt in vielen Stoffwechselvorgängen wichtige Funktionen, insbesondere als Bestandteil vieler Proteine, wie z.B. des Bindegewebsproteins Kollagen, als Komponente zur Synthese des Häm oder in der Funktion als Neurotransmitter im Zentralnervensystem.

Glycin wird z.B. in Nahrungsergänzungsmitteln oder in biochemischen Puffersystemen eingesetzt. Eine häufige Verwendung findet Glycin auch als Komponente in Ernährungsformulierungen zur Anwendung in der Intensivmedizin sowie in biotechnologischen Nährmedien. Glycin wird für diese Anwendungsgebiete häufig in großen Mengen benötigt.
Zur industriellen Weiterverarbeitung muss das Glycin frei rieselnd vorliegen damit es aus den Transportbehältnissen leicht zu entnehmen und auf den Maschinen zur Herstellung der gewünschten Formulierungen problemlos zu bearbeiten ist. In den Gebinden - unabhängig von der Größe und der Art - kommt es jedoch immer wieder zu solch starken Verklumpungen oder gar kompletten Verbackungen (Monoblockbildung) des Materials, so dass es nicht mehr frei fließend aus den Behältnissen entnommen werden kann. Dies führt bei den Anwendern zu erheblichen Problemen im Produktionsablauf, da das verbackene Material erst wieder mechanisch zerkleinert werden muss (was bei einigen Gebindearten praktisch nicht mehr möglich ist). Diese Verbackungen erfolgen teilweise schon nach wenigen Wochen Lagerung.

Die Patentschrift DE 26 22 462 A1 offenbart ein Verfahren zur Druckverformung von körnigen Feststoffen zu einstückig geformten Pellets oder Tabletten, wobei mindestens 0,01 Gew.-% körniges festes Kollagenprotein gemahlen und mit den durch Druck zu verformenden körnigen Feststoffen vermischt wird.

Die Patentschrift DE 697 06 988 T2 offenbart ein Verfahren zur Herstellung verdichteter Partikel aus einer partikulären pharmazeutischen Präparation, umfassend das Kompaktieren der Präparation zum Erhalt einer kompaktierten pharmazeutischen Präparation und das Größenreduzieren der kompaktierten Präparation zu verdichteten Partikeln einer geeigneten Größe und Dichte für medizinische Anwendungen. Glycin ist nur als "Transportsubstanz" für die Arzneistoffe oder pharmazeutischen Mittel erwähnt und nicht als Hauptkomponente der Zusammensetzung.

Aufgabe der vorliegenden Erfindung war es daher, einen Weg zu finden, Glycin-Pulver in einer lagerstabilen und rieselfähigen Form zur Verfügung zu stellen.

Es wurde gefunden, dass die Partikelgröße des Glycin-Pulvers einen sehr starken Einfluss auf dessen Lagerstabilität hat. Glycin mit einer Partikelgröße von mindestens 0,7 mm zeigt auch bei langer Lagerung kaum Verbackungen. Es bleibt wesentlich länger rieselfähig als Pulver mit geringerer Partikelgröße. Zudem ist die Auflösegeschwindigkeit in Wasser trotz der größeren Partikelgröße im Vergleich zur Pulverware ungefähr gleich groß.

Gegenstand der vorliegenden Erfindung ist daher ein Glycin - Granulat, wobei mindestens 75% (w/w) des Glycin - Granulats eine Korngröße von mindestens 0,7 mm aufweist. In einer bevorzugten Ausführungsform weisen 85%, besonders bevorzugt 90%, insbesondere weisen 95% des Granulats eine Korngröße von mindestens 0,7 mm auf.

In einer bevorzugten Ausführungsform weisen 80%, besonders bevorzugt 90%, insbesondere 95% des Granulats eine Korngröße von mindestens 0,8 mm auf.

In einer besonders bevorzugten Ausführungsform weisen 80%, besonders bevorzugt 90%, insbesondere 95% des Granulats eine Korngröße von mindestens 1 mm auf.

In einer bevorzugten Ausführungsform hat das Glycin - Granulat eine Schüttdichte kleiner oder gleich 0,9 g/ml, bevorzugt zwischen 0,5 und 0,8 g/ml. In einer bevorzugten Ausführungsform hat das Glycin - Granulat eine Stampfdichte kleiner oder gleich 1 g/ml, bevorzugt zwischen 0,6 und 0,9 g/ml.

In einer weiteren bevorzugten Ausführungsform hat das Glycin Granulat einen Trocknungsverlust von nicht größer als 0,3%, bevorzugt nicht größer als 0,2%, besonders bevorzugt nicht größer als 0,1%.

In einer bevorzugten Ausführungsform ist das Glycin - Granulat nach Lagerung über 3, bevorzugt 6, besonders bevorzugt 12 Monate in einem verschlossenen Gebinde bei Raumtemperatur rieselfähig. Die Luftfeuchte der Umgebung beträgt während der Lagerung bevorzugt zwischen 20 und 30% r.F..

Gegenstand der vorliegenden Erfindung ist auch ein Glycin - Granulat, wobei mindestens 75% (w/w) des Glycin - Granulats eine Korngröße von mindestens 0,7 mm aufweist, herstellbar durch Kompaktierung.

In einer bevorzugten Ausführungsform weisen 85%, besonders bevorzugt 90%, insbesondere weisen 95% des Granulats eine Korngröße von mindestens 0,7 mm auf.

In einer bevorzugten Ausführungsform weisen 80%, besonders bevorzugt 90%, insbesondere 95% des Granulats eine Korngröße von mindestens 0,8 mm auf.

In einer besonders bevorzugten Ausführungsform weisen 80%, besonders bevorzugt 90%, insbesondere 95% des Granulats eine Korngröße von mindestens 1 mm auf.

Die Prozentangaben beziehen sich dabei jeweils auf die Masse des Granulats (w/w).

In einer bevorzugten Ausführungsform erfolgt die Herstellung durch
a) Bereitstellen von Glycin
b) Kompaktieren des Glycins aus Schritt a) in einem Rollenkompaktor. Typischerweise ist das bereitgestellte Glycin pulverförmig. Bevorzugt liegt die Korngröße von mindestens 75% (w/w) der Pulverteilchen unterhalb von 0,7 mm.

In einer bevorzugten Ausführungsform ist in Schritt b) die Presskraft des Rollenkompaktors zwischen 1 und 50 KN/cm Rollenweite.

In einer besonders bevorzugten Ausführungsform erfolgt die Herstellung durch
a) Bereitstellen von Glycin
b) Kompaktieren des Glycins aus Schritt a) in einem Rollenkompaktor, wobei ein Kompaktat entsteht, das Partikel mit einer Korngröße über 0,7 mm enthält.
c) Zumindest teilweise Rückführung von Glycin-Partikeln aus dem in Schritt b) erhaltenen Kompaktat, die eine Korngröße kleiner 0,7 mm haben, zu dem in Schritt a) bereitgestellten Glycin.

In einer weiteren bevorzugten Ausführungsform erfolgt die Rückführung in Schritt c) durch Brechung der Kompaktate und Klassierung nach Korngröße, wobei Glycin-Partikeln, die eine Korngröße kleiner 0,7 mm haben, zumindest teilweise zu dem in Schritt a) bereitgestellten Glycin rückgeführt werden.

Gegenstand der vorliegenden Erfindung ist auch ein Verfahren zur Herstellung von Glycin - Granulat, wobei mindestens 75% (w/w) des Glycin-Granulats eine Korngröße von mindestens 0,7 mm aufweist, durch
a) Bereitstellen von Glycin - Pulver
b) Kompaktieren des Glycin - Pulvers aus Schritt a).

In einer bevorzugten Ausführungsform erfolgt das Kompaktieren in einem Rollenkompaktor.

In einer besonders bevorzugten Ausführungsform erfolgt die Herstellung durch
a) Bereitstellen von Glycin Pulver
b) Kompaktieren des Glycin - Pulvers aus Schritt a) in einem Rollenkompaktor, wobei ein Kompaktat entsteht, das Glycin-Partikel mit einer Korngröße über 0,7 mm, bevorzugt über 1mm, enthält.
c) Zumindest teilweise Rückführung von Glycin-Partikeln aus dem in Schritt b) erhaltenen Kompaktat, die eine Korngröße kleiner 0,7 mm haben, zu dem in Schritt a) bereitgestellten Glycin-Pulver.

In einer bevorzugten Ausführungsform ist in Schritt b) die Presskraft des Rollenkompaktors zwischen 1 und 50 KN/cm Rollenweite.

In einer weiteren bevorzugten Ausführungsform erfolgt die Rückführung in Schritt c) durch Brechung der Kompaktate und Klassierung nach Korngröße, wobei Glycin-Partikeln, die eine Korngröße kleiner 0,7 mm, haben, zumindest teilweise zu dem in Schritt a) bereitgestellten Glycin rückgeführt werden.

Gegenstand der Erfindung ist dementsprechend auch Glycin-Granulat herstellbar nach dem erfindungsgemäßen Verfahren.

Gegenstand der vorliegenden Erfindung ist auch die Verwendung von Glycin-Granulat, wobei mindestens 75% (w/w) des Glycin-Granulats eine Korngröße von mindestens 0,7 mm aufweist, insbesondere des erfindungsgemäß kompaktierten Glycin-Granulats, zur Herstellung von Nährmedien für medizinische Anwendungen oder in der Biotechnologie. Zusätzlich zu den oben aufgeführten einzelnen Ausführungsformen kann die Erfindung auch in jeder Kombination von zwei oder mehreren der oben und im folgenden genannten Ausführungsformen ausgeübt werden.

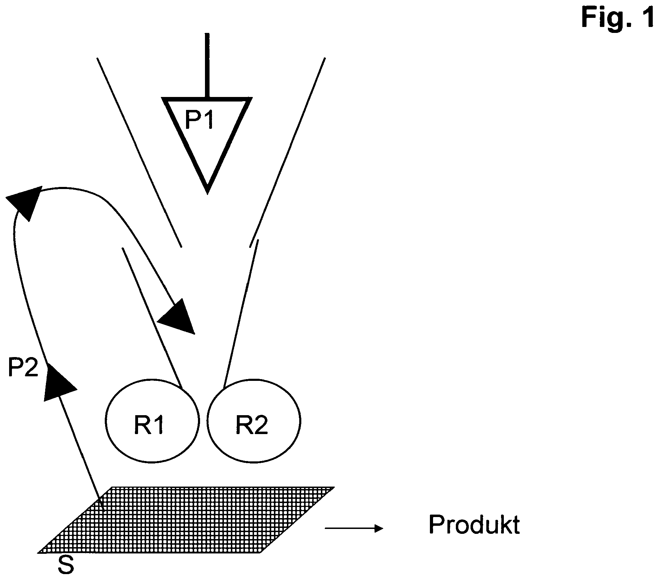

Abbildung 1 zeigt schematisch eine mögliche Anordnung zur Durchführung des erfindungsgemäßen Verfahrens.

Kompaktierung bzw. Kompaktieren von Feststoffen bedeutet erfindungsgemäß die Herstellung von im Verhältnis zum Ausgangsmaterial größeren Partikeln eines Feststoffs durch Zusammenpressen des Ausgangsmaterials unter Druck. Das Kompaktieren erfolgt ohne Zugabe von Wasser oder anderen Lösungsmitteln. Es handelt sich um eine Trockenkompaktierung. Die Kompaktierung kann beispielsweise in Rollenkompaktoren, Excentertablettenpressen oder Rundläuferpressen erfolgen. Das Kompaktieren erfolgt erfindungsgemäß bevorzugt in Rollenkompaktoren.

Rollenkompaktoren werden auch Rollenpressen oder Walzenpressen genannt. Dies sind Pressmaschinen mit zwei bevorzugt gegenläufigen Walzen. Die Walzen haben einen bestimmten Abstand voneinander und bilden somit einen Spalt. Durch diesen Spalt wird das zu kompaktierende Material gepresst. Spaltbreite und Länge der Rollen bzw. des entstehenden Spaltes variieren je nach Modell. Weiterhin können die Rollen vertikal, horizontal oder geneigt angeordnet sein. Die Rollen können ein Profil aufweisen oder bevorzugt eine glatte oder geriffelte Oberfläche haben. Die Zufuhr des zu kompaktierenden Materials in den Rollenkompaktor kann beispielsweise mittels Schwerkraft oder durch Schnecken erfolgen. Erfindungsgemäß geeignete Rollenkompaktoren sind beispielsweise von den Firmen Alexanderwerk, Sahut Conreur, Hosokawa oder Fitzpatrick Company erhältlich.

Als Klassieren wird erfindungsgemäß das Trennen eines dispersen Feststoffgemisches in Fraktionen nach der Partikelgröße bezeichnet. Bevorzugt erfolgt das Klassieren mittels Sieben. Alternative Klassierungsmethoden sind Windsichtung oder Vibration. Das Ergebnis einer Klassierung sind mindestens zwei Fraktionen, die sich dadurch unterscheiden, dass die Mindestgrenze der Partikelgröße der einen Fraktion zugleich die Höchstgrenze der anderen Fraktion ist. Feststoffpartikel, die genau dazwischen liegen, nennt man Grenzkorn. Dies ist allerdings eine idealisierte Betrachtung des Trennvorgangs. In der Praxis, insbesondere bei durch Siebung klassierten Pulvern, existieren mehr oder weniger große Übergangsbereiche zwischen den Klassen.

Sieben ist ein mechanisches Trennverfahren zur Größentrennung (Klassieren) von Schüttgütern bzw. dispersen Feststoffgemischen. Das zu trennende Material wird dazu auf ein Sieb gegeben, das z.B. in Rotation versetzt oder geschüttelt wird. Die Triebkraft für das Sieben ist in der Regel die Gewichtskraft. Um Partikel möglichst oft mit dem Sieb in Kontakt zu bringen, wird durch Schwingen, Vibrieren und/oder Taumeln das zu trennende Gut bewegt. Das Sieben ist bestimmt durch die Durchtrittswahrscheinlichkeit eines Korns bei einer gegebenen Maschenweite. Technisch wird dies zum Beispiel mit einem Plansichter, einer Taumelsiebmaschine oder Vibrationssiebmaschine ausgeführt. Oft sind die Siebe in mehreren Sieblagen übereinander angeordnet.

Die Korngröße eines Partikels wird durch Siebung ermittelt. Hierbei wird ein Satz mit nach unten immer feiner werdenden Sieben aufeinander gesetzt. Die zu analysierende Probe wird in das oberste Sieb eingefüllt und der Siebsatz anschließend in eine Siebmaschine eingespannt. Die Maschine rüttelt oder vibriert dann den Siebsatz für einen gewissen Zeitraum mit einer gewissen Amplitude. Gelangt ein Partikel beispielsweise nicht durch ein Sieb mit einer Maschenweite von 0,7 mm, so hat der Partikel eine Korngröße von größer 0,7 mm.

Das Ergebnis einer Korngrößenanalyse mittels Siebung ist die Korngrößenverteilung, also eine Häufigkeitsverteilung. Daraus lassen sich die üblichen statistischen Parameter, wie Mittelwert, Median, Perzentilwerte, Streuung oder Schiefe der Verteilung berechnen und damit die Probe bezüglich ihrer Korngröße charakterisieren. Eine Korngrößenverteilung kann eng sein oder breit, in Abhängigkeit davon, wie groß bei einer Probe die Unterschiede in der Korngröße sind.

Pulver sind Haufwerke aus festen Partikeln. Die einzelnen Partikel unterscheiden sich durch Größe, Form, Masse und Oberfläche. Der Zusammenhalt wird durch Kohäsionskräfte gewährleistet. Pulver können Kristalle, amorphe Substanzen, Aggregate oder Agglomerate sein. Erfindungsgemäß werden als Glycin-Pulver Mischungen von Glycin-Teilchen bezeichnet, von denen weniger als 75% (w/w) eine Korngröße von mindestens 0,7 mm aufweisen.

Granulate bestehen aus Körnern bzw. Partikeln. Die erfindungsgemäßen Granulate bestehen aus Körnern bzw. Partikeln, von denen mindestens 75% (w/w) eine Korngröße von mindestens 0,7 mm aufweisen. Bevorzugt bestehen die erfindungsgemäßen Granulate aus Partikeln bzw. Körnern, die ein Agglomerat aus Pulverpartikeln sind. Das bedeutet, die Granulat-Partikel sind durch Kompaktierung von Pulver-Partikeln entstanden. Typischerweise haben Granulat-Partikel eine asymmetrische Form.

Als verbacken wird erfindungsgemäß ein Pulver bezeichnet, dessen Partikel nicht mehr frei gegeneinander beweglich sind und das daher nicht mehr rieselfähig bzw. frei fließend ist. Dieses Merkmal wird typischerweise visuell bestimmt. Während leicht verbackene Pulver gegebenenfalls als größere Klumpen noch gegeneinander beweglich sind, bilden stark verbackene Pulver zumeist eine Art monolithischen Block. Klumpen oder Block müssen gegebenenfalls mechanisch zerstört werden, um wieder ein frei fließendes Pulver zu erhalten.

Ein rieselfähiges bzw. frei fließendes Pulver kann beispielsweise gleichmäßig ohne größere Klumpen aus einem Gefäß geschüttet werden.

Lagerstabil ist ein Granulat, wenn es über die Zeit seiner Lagerung seine Eigenschaften nicht wesentlich verändert. Beispielsweise ist Glycin-Granulat lagerstabil, wenn es während der Zeit der Lagerung rieselfähig bleibt.

Es wurde gefunden, dass die Eigenschaften des Feststoffes Glycin durch Veränderung der Partikelgröße stark beeinflusst werden können. Insbesondere kann das Verbacken beeinflusst werden. Während Glycin in Form von feinen Partikel mit einer Korngröße unter 0,5 mm, insbesondere unter 0,1 mm, häufig selbst unter Feuchtigkeitsausschluss zu unkontrollierten Verbackungen neigt, wird dies durch Kompaktierung der feinen Partikel zu größeren Kompaktat-Partikeln mit einer Korngröße von über 0,7 mm weitgehend verhindert. Dabei werden die Partikel neben dem Verpressen bei der Kompaktierung und dem anschließenden optionalen Aufbrechen der Schülpen (Granulieren) und Klassieren bevorzugt keinem weiteren Behandlungsschritt unterzogen. Die Kompaktierung erfolgt ohne Zusatz von Reagenzien, wie Wasser oder anderen Lösungsmitteln.

Daher erfolgt das erfindungsgemäße Verfahren zur Herstellung von kompaktiertem Glycin typischerweise durch
a) Bereitstellen von Glycin, bevorzugt Glycin-Pulver. Dieses Glycin-Pulver besteht aus Partikeln, von denen weniger als 75% (w/w) eine Korngröße von mindestens 0,7 mm aufweisen. In der Regel haben mindestens 65% (w/w) der Teilchen des Pulvers eine Korngröße von höchstens 0,5 mm. Als Ausgangsmaterial geeignetes pulverförmiges Glycin ist beispielsweise Glycin der Firma Merck KGaA, Deutschland, mit der Artikelnummer 100590.
b) Kompaktieren des pulverförmigen Glycins aus Schritt a)

Das Kompaktieren, d.h. das Trockenverpressen, erfolgt bevorzugt in einem Rollenkompaktor. Rollenkompaktoren sind dem Fachmann bekannt und kommerziell erhältlich. Erfindungsgemäß besonders geeignet sind Rollenkompaktoren mit einem Abstand zwischen den Rollen von 0,5 bis 3 mm, bevorzugt zwischen 1 und 2 mm. Erfindungsgemäß besonders geeignete Rollenkompaktoren haben Rollen mit einer Breite zwischen 10 und 50 cm, so dass der Spalt zwischen den beiden Rollen eine Länge zwischen 10 und 50 cm hat.

Die Presskraft des Rollenkompaktors liegt typischerweise zwischen 0,1 und 100 kN/cm Rollenbreite. Bevorzugt werden die Rollen mit einer Kraft zwischen 1 und 50 kN zusammengepresst.

Die Zuführung des bereitgestellten Glycins in den Rollenkompaktor erfolgt bevorzugt mittels einer oder mehrerer Schnecken. Rollenkompaktoren erzeugen durch Verpressen von Pulvern zwischen bevorzugt gegenläufig rotierenden Walzen größere Kompaktate der Pulver. Bei Verwendung von Rollen mit glatten oder geriffelten Oberflächen erhält man häufig platten-, schülpen- und/oder schollenförmige Kompaktate.

Die Form und Größe der aus dem Rollenkompaktor erhaltenen Kompaktate ist typischerweise sehr unregelmäßig und kann durch die weitere Verarbeitung beeinflusst werden. Allein das Umfüllen oder Verpacken der Kompaktate wird typischerweise zu einem teilweisen Zerbrechen der Schülpen führen.

Die direkt aus dem Rollenkompaktor erhaltenen Kompaktate werden deshalb bevorzugt in einem weiteren Verfahrensschritt zu Granulaten bzw. größeren Partikeln aufgebrochen. Dies kann beispielsweise durch einen Walzenbrecher mit anschließender Siebung über ein vibrierendes Sieb oder Reibsieb, durch eine Rotorhomogenisierungsmühle oder eine Oszilliermühle (oszillierendes Schwingsieb) erfolgen. Aus den unregelmäßigen, schollenförmigen Kompaktaten werden so Granulat-Partikel aus verpresstem Pulver. Die Korngröße der Partikel kann durch die Maschenweite des Siebes eingestellt werden. Auf diese Weise kann auch die Obergrenze der Korngröße beeinflusst werden. Bevorzugt werden Granulate erzeugt, deren Korngröße nicht oberhalb von 5 mm liegt.

Weiterhin werden die Kompaktate oder die zu kompaktierten Partikeln aufgebrochenen Kompaktate anschließend bevorzugt klassiert. Dadurch kann ein bezüglich der Korngröße homogeneres Material erhalten werden und zusätzlich bevorzugt der Feinanteil zumindest teilweise abgetrennt werden. Als Feinanteil werden erfindungsgemäß Partikel bezeichnet, die eine Korngröße unter der gewünschten Mindestkorngröße von z.B. 0,7 mm, 0,8 mm oder 1 mm haben.

Je nach Durchführung der Kompaktierung und/oder des Aufbrechens können Granulate mit einem geringen oder einem hohen Feinanteil entstehen. Bei Granulaten mit einem geringen Feinanteil, ist es typischerweise nicht nötig, diesen abzutrennen. Ist dies jedoch gewünscht oder notwendig, um die Produktspezifikation bezüglich der Korngrößenanteile zu erfüllen, kann eine Klassierung durchgeführt werden. Um Materialverlust zu vermeiden und den Feinanteil wiederzuverwerten, kann dieser abgetrennt und erneut dem Material zugefügt werden, das der Kompaktierung unterzogen wird.

Durch die Klassierung und die Durchführung der Rückführung kann bestimmt werden, welcher Anteil der Partikel rückgeführt wird. Werden bei der Klassierung Partikel mit einer Korngröße unter z.B. 0,7 mm abgetrennt, können diese alle rückgeführt werden. Man kann jedoch auch weitere Untergruppen erzeugen und zwar die Partikel mit einer Korngröße von z.B. unter 0,7 mm vom Produkt abtrennen, diese aber nicht komplett rückführen sondern beispielsweise nur Partikel unter 0,5 mm oder unter 0,2 mm. Genauso kann die Klassierung so durchgeführt werden, dass nur Partikel mit einer Korngröße von z.B. 0,5 mm oder 0,2 mm abgetrennt und rückgeführt werden, so dass das Produkt noch einen Anteil an Partikeln mit einer Korngröße von 0,5 mm bzw. 0,2 mm bis z.B. 0,7 mm enthält - sofern dieser Anteil nicht den für das entsprechende Produkt definierten maximalen Feinanteil überschreitet.

In einer bevorzugten Ausführungsform des erfindungsgemäßen Verfahrens wird der Feinanteil ganz oder teilweise durch Klassierung aus dem Produkt entfernt und bevorzugt vollständig kontinuierlich wieder dem in Schritt a) bereitgestellten Material gemischt und so erneut dem Kompaktierungsschritt zugeführt. Diese Rückführung reduziert den Feinanteil im gewünschten kompaktierten Produkt, ohne dass Materialverlust in Kauf genommen werden muss.

Es wurde gefunden, dass Granulate des Glycins eine wesentlich geringere Neigung zum Verbacken haben, wenn sie aus Partikeln bestehen, von denen mindestens 75% (w/w) eine Korngröße von mindestens 0,7 mm aufweisen. Bevorzugt wurden die Granulate mittels Kompaktierung hergestellt. Besonders bevorzugt haben sie einen Feinanteil von unter 20%, besonders bevorzugt unter 10% (w/w).

Abbildung 1 zeigt schematisch eine mögliche Anordnung zur Durchführung des erfindungsgemäßen Verfahrens. Der Rollenkompaktor ist dargestellt mit den Rollen R1 und R2. Das Glycin-Pulver P1 wird aus einem Reservoir mittels Schwerkraft dem Rollenkompaktor zugeführt. Alternativ kann dazu auch eine in Abbildung 1 nicht dargestellte Transportschnecke verwendet werden. Das kompaktierte Produkt, das aus dem Rollenkompaktor freigesetzt wird, wird mittels einer Mahl- und Siebvorrichtung, dargestellt als Sieb S, aufgebrochen und klassiert. Die gewünschte Produktfraktion wird zur weiteren Verwendung und ggf. Verpackung entfernt und der Feinanteil P2, der eine Korngröße unterhalb von z.B. 0,7 mm aufweist wird kontinuierlich wieder dem pulverförmigen Glycin zugeführt, das in den Rollenkompaktor gefüllt wird.

Gegenstand der vorliegenden Erfindung ist auch Glycin-Granulat, wobei mindestens 75% (w/w) des Glycin-Granulats eine Korngröße von mindestens 0,7 mm aufweist.

Bevorzugt wird dieses Glycin mittels Kompaktierung hergestellt. Im Folgenden wird dieses Glycin daher auch kompaktiertes Glycin-Granulat genannt. Bevorzugt hat dieses Glycin einen Feinanteil von unter 20%, besonders bevorzugt unter 10% (w/w).

Bevorzugt ist dieses kompaktierte Glycin-Granulat erhältlich durch das erfindungsgemäße Verfahren.

In einer bevorzugten Ausführungsform hat das kompaktierte Glycin-Granulat eine Schüttdichte kleiner oder gleich 0,9 g/ml, bevorzugt zwischen 0,5 und 0,8 g/ml.

In einer bevorzugten Ausführungsform hat das kompaktierte Glycin-Granulat eine Stampfdichte kleiner oder gleich 1 g/ml, bevorzugt zwischen 0,6 und 0,9 g/ml.

In einer weiteren bevorzugten Ausführungsform hat das kompaktierte Glycin-Granulat einen Trocknungsverlust von nicht größer als 0,3%, bevorzugt nicht größer als 0,2%, besonders bevorzugt nicht größer als 0,1%.

In einer weiteren Ausführungsform hat das kompaktierte Glycin-Granulat einen Hausner-Faktor von kleiner oder gleich 1,18 und einen Kompressibilitätsindex von kleiner oder gleich 15%.

Insbesondere zeigte sich überraschend, dass die Lagerstabilität des kompaktierten Glycin-Granulats wesentlich besser ist als die des pulverförmigen Ausgangsmaterials. Während das pulverförmige Ausgangsmaterial schon nach wenigen Wochen verbackt und dadurch nicht mehr rieselfähig ist, verbackt das kompaktierte Glycin-Granulat wesentlich geringer bzw. wesentlich später. Beispielsweise verbackt das kompaktierte Glycin-Granulat bei einer offenen Lagerung bei 25°C und 60% r.F. nach 7 Wochen überhaupt nicht, während das pulverförmige Material schon nach einer Woche verbackt. Weitere Vergleiche unter unterschiedlichen Lagebedingungen finden sich in den Beispielen.

Es wurde gefunden, dass das kompaktierte Glycin-Granulat nach Lagerung über 3, bevorzugt 6, besonders bevorzugt 12 Monate in einem verschlossenen Gebinde bei Raumtemperatur rieselfähig bleibt. Die Luftfeuchte der Umgebung beträgt während der Lagerung zwischen 20 und 30% r.F.. Bevorzugt ist das Gebinde luftdicht verschlossen. Auf diese Weise kann die Lagerstabilität gegebenenfalls noch weiter erhöht werden. Ein Gebinde ist jede Art von Verpackung, die für die Lagerung von Pulvern oder Granulaten geeignet ist und verschlossen werden kann. Dem Fachmann sind derartige Gebinde bekannt. Bevorzugte Gebinde sind Schraubdeckelbehälter aus Glas oder Kunststoff, wie z.B. aus PE, PE-Säcke, big bags oder Kunststoff-Fässer.

Dies ist insbesondere überraschend, da Glycin sowohl als Pulver wie auch als Kompaktat wenig hygroskopisch ist und kompaktiertes Glycin nicht weniger hygroskopisch ist (siehe Versuche zur Hygroskopizität).

Weiterhin zeigt das kompaktierte Glycin-Granulat ein ähnliches Lösungsverhalten wie pulverförmiges Glycin. Das bedeutet, kompaktiertes Glycin-Granulat kann unter denselben Bedingungen genauso schnell in Lösung gebracht werden wie pulverförmiges, nicht kompaktiertes Glycin. Gegenstand der vorliegenden Erfindung ist auch die Verwendung des kompaktierten Glycin-Granulats zur Herstellung von Nährmedien für medizinische Anwendungen oder in der Biotechnologie. Beispiele für derartige Nährmedien sind insbesondere Injektionslösungen, Infusionslösungen und Zellkulturmedien. Derartige Nährmedien liegen typischerweise als Feststoffgemisch oder wässrige Lösung vor. Das kompaktierte Glycin-Granulat kann den Medien als Feststoff zugesetzt werden oder in Wasser oder einem anderen Lösungsmittel gelöst und dann den Nährmedien zugesetzt werden. Verfahren zur Herstellung von Nährmedien sind dem Fachmann bekannt. Bevorzugt werden die Komponenten des Nährmediums in fester Form vermischt und vermahlen. Anschließend werden sie durch Zugabe von Wasser oder einem wässrigen Puffer gelöst. Während hygroskopische oder instabile Komponenten gegebenenfalls separat gelagert und in Lösung gebracht werden müssen, kann das kompaktierte Glycin-Granulat problemlos mit den übrigen stabilen Komponenten verarbeitet werden.

Gegenstand der vorliegenden Erfindung ist auch ein Verfahren zur Erhöhung der Lagerstabilität von Glycin-Pulver, insbesondere zum Erhalt der Rieselfähigkeit von Glycin. Dabei wird Glycin-Pulver zu Glycin-Granulat kompaktiert, wobei mindestens 75% (w/w) des erhaltenen Glycin-Granulats eine Korngröße von mindestens 0,7 mm aufweist.

Dabei gelten die vorab genannten bevorzugten Ausführungsformen des Herstellverfahrens von kompaktiertem Glycin-Granulat. Das Verfahren kann bei Raumtemperatur durchgeführt werden. Das mittels des Verfahrens erhaltene Glycin-Granulat weist eine deutlich höhere Lagerstabilität auf als das als Ausgangsmaterial eingesetzte Glycin-Pulver. Insbesondere bleibt die Rieselfähigkeit im Vergleich zu Glycin-Pulver bei gleichen Lagerbedingungen länger erhalten.

Die erfindungsgemäßen Verfahren sind einfach und effektiv in der Durchführung. Ohne chemische Veränderung des Glycins, rein durch mechanische Einwirkungen wie Verpressen und optional Sieben, können die Eigenschaften des Glycin verändert werden. Insbesondere erfolgt eine deutliche Erhöhung der Lagerstabilität, da das erfindungsgemäße Material wesentlich langsamer verbackt und länger rieselfähig bleibt.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### A) Geräte und Verfahren zur Charakterisierung der Stoffeiqenschaften

1.Schüttdichte: gemäß DIN EN ISO 60: 1999 (Deutsche Fassung)
   - Angabe in "g/ml"
2.Stampfdichte: gemäß DIN EN ISO 787-11: 1995 (Deutsche Fassung)
   - Angabe in "g/ml"
3.Kompressibiltätsindex: gemäß 2.9.36. Powder Flow Ph Eur 8.0 (Englische Fassung)
   - Angabe in "%"
4.Hausner-Faktor: gemäß 2.9.36. Powder Flow Ph Eur 8.0 (Englische Fassung)
   - keine Dimension
5.Partikelgrößenbestimmung durch Trockensiebung über einen Siebturm: Retsch AS 200 control, Fa.Retsch (Deutschland); Substanzmenge: ca. 110,00 g; Siebzeit: 30 Minuten; Amplitudenintensität: 1mm; Intervall: 5 Sekunden; Analysensiebe mit Metalldrahtgewebe gemäß DIN ISO 3310
   - Siebweiten (in µm) für Vergleich 1 und 2: 1000, 710, 600, 500, 400, 355, 300, 250, 200, 150, 100, 50, 32
   - Siebweiten (in µm) für Beispiel A, B, C, D, E und F: 2000, 1700, 1600, 1400, 1250, 1120, 1000, 900, 800, 710, 600, 500
   - Angabe der Mengenverteilung pro Siebfraktion in den Tabellen als "Gew.% der Einwaage"
6.Bestimmung des Trocknungsverlusts:
   Bestimmung nach Ph Eur 8.6 gemäß Glycin Monographie: 1,000 g
   Substanz bei 105 °C 2 Stunden im Ofen (Ph Eur 8.6 unter 2.2.32.) trocknen
      - Angabe in "Gew. %"
7.DVS-Bedingungen (Bestimmung der Hygroskopizität):
   Surface Measurement Systems Ltd. UK 1996 - 2007, Method: 0-98 % r.F.,
   10% Steps, 25° C, 0,0005wt%-min., Halfcycle.sao; Durchführung der
   Messung gemäß Herstellervorgaben
      - Angabe der Gewichtszunahme in "Gew. %"
8. Lösungsgeschwindigkeit:
   Verwendete Geräte: Waage Mettler AT201, Becherglas 150 mL,
   Magnetrührplatte IKA® RCT basic, Messzylinder 50 ml, Rührfisch Durchmesser 7 mm und Länge 4 cm, Rührgeschwindigkeit 200 Upm,
   Substanzmenge 4,00g+/-0,1g, VE-Wasser mit einer Temperatur zwischen 20 und 25°C ("VE-Wasser" ist "voll entsalztes Wasser");
   Durchführung: 50mL VE-Wasser in Becherglas geben und Rührer einschalten. Thermometer in VE-Wasser platzieren, Substanz hinzugeben und Messung der Zeit (Stoppuhr) bis die Substanz visuell rückstandsfrei gelöst vorliegt.
      - Angabe in "Sekunden"
9.Lagerbedingungen in Klimaschränken:
   a) Vergleiche 1 und 2 sowie die Beispiele E und F: je 160g+/-5g Substanz werden bei 25°C/60%r.F. sowie 40°C/75%r.F sowohl offen (in einer Glasschale) als auch geschlossen (in einem Schraubdeckelglas) gelagertdie Beurteilung der Verbackung erfolgt nach 1, 2 und 7 Wochen Lagerzeit.
      - Glasschale: 95mm Durchmesser, 55mm Höhe, die Substanz wird in gleichmäßiger Schichtdicke über den Schalenboden verteilt; die Beurteilung erfolgt durch Neigung der Schale und einer visuellen Beobachtung des Fließverhaltens je nach Neigungswinkel (teilweise sind die Muster jedoch bereits so stark verbacken dass kein freies Pulverfließen mehr beobachtet werden kann)
      - Schraubdeckelglas: 250ml, Höhe 11,5cm, Außendurchmesser 7cm, Weißglas mit Kunststoffschraubdeckel fest verschlossen, die Beurteilung erfolgt durch Neigung des Glases und einer visuellen Beobachtung des Fließverhaltens je nach Neigungswinkel (teilweise sind die Muster jedoch bereits so stark verbacken dass kein freies Pulverfließen mehr beobachtet werden kann - nach Drehung des Glases um 180° bleibt das Glycin in diesen Fällen am Glasboden haften bzw. fällt nur teilweise ab)
   b) Beispiele A, B. C und D: je 120g+/-5g Substanz werden bei 40°C/75%r.F sowohl offen (in einer Glasschale) als auch geschlossen (in einem Schraubdeckelglas) gelagert - die Beurteilung der Verbackung erfolgt nach 2 und 7 Wochen Lagerzeit.
      - Glasschale: 95mm Durchmesser, 55mm Höhe, die Substanz wird in gleichmäßiger Schichtdicke über den Schalenboden verteilt; die Beurteilung erfolgt durch Neigung der Schale und einer visuellen Beobachtung des Fließverhaltens je nach Neigungswinkel (teilweise sind die Muster jedoch bereits so stark verbacken, dass kein freies Pulverfließen mehr beobachtet werden kann)
      - Schraubdeckelglas: 250ml, Höhe 11,5cm, Außendurchmesser 7cm, Weißglas mit Kunststoffschraubdeckel fest verschlossen, die Beurteilung erfolgt durch Neigung des Glases und einer visuellen Beobachtung des Fließverhaltens je nach Neigungswinkel (teilweise sind die Muster jedoch bereits so stark verbacken dass kein freies Pulverfließen mehr beobachtet werden kann - nach Drehung des Glases um 180° bleibt das Glycin in diesen Fällen am Glasboden haften bzw. fällt nur teilweise ab)
10.Gewichtsänderungen nach Lagerung
   - Angabe der Gewichtsänderung in "g" im Vergleich zum Startwert bei Einlagerungsbeginn
11."Break Energy" Bestimmung:
   REVOLUTION Powder Analyzer (Mercury Scientific Inc, Newton, USA);
   Rotation Rate 0,3rpm, Test Method FlowMethod_SP.fam; 100 mm Trommeldurchmesser; 95-100 ml Pulvermenge
   -Angabe in "mJ"
12."Avalanche Angle" Bestimmung:
   REVOLUTION Powder Analyzer (Mercury Scientific Inc, Newton, USA);
   Rotation Rate 0,3rpm, Test Method FlowMethod_SP.fam; 100 mm Trommeldurchmesser; 95-100 ml Pulvermenge
   -Angabe in Grad ("°")

### B) Arbeitsmethoden

Handelsübliches kristallines pulverförmiges Glycin (Artikel 100590 Glycin krist. geeignet zur Verwendung als Excipients EMPROVE® exp Ph Eur, BP, JP, USP der Firma Merck KGaA, Darmstadt, Deutschland) wird einer Trockengranulierung über Walzenkompaktoren mit anschließender Brechung und Siebung über eine Oszillatorsiebmühle unterworfen.

Es wird mit zwei verschiedenen Kompaktoren gearbeitet:
1. Beipiele A bis D: Kompaktor Typ RC 100 der Fa. powtec Maschinen und Engineering, Remscheid, Deutschland; Walzendurchmesser 100 mm, Walzenbreite 30 mm, geriffelte Walzenoberfläche, keine Rollenkühlung, Siebmaschenweite 3 mm, Unterkornsiebung 1 mm ohne Materialrückführung; die Spaltbreite ergibt sich durch den gewählten Walzendruck
2.Beispiele E und F: Kompaktor Typ K200/100 mit Siebmühle FC 400 der Fa. Hosokawa Bepex, Leingarten, Deutschland; Walzendurchmesser 200 mm, Walzenbreite 100 mm, geriffelte Walzenoberfläche, keine Rollenkühlung, Siebmaschenweit 2 mm; Unterkornsiebung 1 mm mit Materialrückführung; die Spaltbreite ergibt sich durch den gewählten Walzendruck

Der Vergleich gegen den Stand der Technik erfolgt gegen 2 Chargen eines handelsüblichen kristallinen pulverförmigen Glycins der Firma Merck KGaA, Darmstadt (Deutschland), Artikel 100590 Glycin krist..

### 1.Beispiele A. B. C und D: Herstellung eines kompaktierten Gylcins mit verbesserter Rieselfähigkeit und Lagerstabilität (kompaktiertes Glycin mit Grobanteilen >2000 µm hergestellt unter verschiedenen Walzendrücken)

### Kompaktierung Beispiel A:

Schneckendrehgeschwindigkeit 30 Upm, Walzendrehgeschwindigkeit 5 Upm, Pressdruck an den Druckrollen 5,23 +/- 0,33 KN/cm

### Kompaktierung Beispiel B:

Schneckendrehgeschwindigkeit 30 Upm, Walzendrehgeschwindigkeit 5 Upm, Pressdruck an den Druckrollen 6,54 +/- 0,33 KN/cm

### Kompaktierung Beispiel C:

Schneckendrehgeschwindigkeit 20 Upm, Walzendrehgeschwindigkeit 4 Upm, Pressdruck an den Druckrollen 7,84 +/- 0,33 KN/cm

### Kompaktierung Beispiel D:

Schneckendrehgeschwindigkeit 30 Upm, Walzendrehgeschwindigkeit 5 Upm, Pressdruck an den Druckrollen 3,92 +/- 0,33 KN/cm

### Upm = Umdrehungen pro Minute

### Schüttdichte. Stampfdichte. Carr-Index:

(Details zu den Messverfahren siehe unter Methoden)

| Probe | Schüttdichte (g/ml) | Stampfdichte (g/ml) | Hausner -Faktor | Kompressibilitätsindex (%) | Trocknungsverlust (%) |
|---|---|---|---|---|---|
| Beispiel A | 0,72 | 0,82 | 1,14 | 12,7 | 0,02 |
| Beispiel B | 0,72 | 0,82 | 1,13 | 11,4 | 0,04 |
| Beispiel C | 0,72 | 0,80 | 1,12 | 10,5 | 0,05 |
| Beispiel D | 0,73 | 0,82 | 1,12 | 10,6 | 0,04 |

### Partikelverteilung bestimmt über Turmsiebung:

Angaben in Gew.% (Details zum Messverfahren siehe unter Methoden)

| Probe | <500 µm | 500-600 µm | 600-710 µm | 710-800 µm | 800-900 µm | 900-1000 µm | 1000-1120 µm |
|---|---|---|---|---|---|---|---|
| Beispiel A | 2,4 | 0,5 | 0,9 | 1,9 | 4,9 | 4,0 | 7,0 |
| Beispiel B | 1,2 | 0,2 | 0,6 | 1,5 | 4,2 | 3,8 | 7,1 |
| Beispiel C | 2,2 | 0,7 | 1,3 | 2,3 | 6,1 | 4,8 | 8,6 |
| Beispiel D | 2,2 | 0,5 | 1,0 | 1,8 | 4,7 | 4,0 | 7,2 |

| Probe | 1120-1250 µm | 1250-1400 µm | 1400-1600 µm | 1600-1700 µm | 1700-2000 µm | > 2000 µm |
|---|---|---|---|---|---|---|
| Beispiel A | 8,0 | 8,9 | 15,3 | 7,4 | 22,7 | 16,1 |
| Beispiel B | 8,6 | 9,8 | 15,6 | 7,1 | 22,2 | 18,1 |
| Beispiel C | 10,6 | 11,0 | 14,5 | 6,6 | 19,2 | 12,1 |
| Beispiel D | 7,7 | 8,0 | 15,1 | 6,4 | 22,6 | 18,8 |

### 2.Beispiele E und F: Herstellung eines kompaktierten Gylcins mit verbesserter Rieselfähigkeit und Lagerstabilität (kompaktiertes Glycin mit geringem Grobanteil >2000µm)

### Kompaktierung Beispiel E und F:

Schneckendrehgeschwindigkeit 17 bis 19 Upm,
Walzendrehgeschwindigkeit 21 Upm, Pressdruck an den Druckrollen 9,15 +/- 0,65 KN/cm
Es wurden 2 Muster gezogen und charakterisiert: Beispiel E und Beispiel F

### Schüttdichte. Stampfdichte. Hausner-Faktor, Kompressibilitätsindex:

(Details zu den Messverfahren siehe unter Methoden)

| Probe | Schüttdichte (g/ml) | Stampfdichte (g/ml) | Hausner-Faktor | Kompressibilitätsindex (%) | Trocknungsverlust (%) |
|---|---|---|---|---|---|
| Beispiel E | 0,72 | 0,80 | 1,12 | 10,6 | 0,03 |
| Beispiel F | 0,72 | 0,81 | 1,12 | 10,4 | 0,03 |

### Partikelverteilung bestimmt über Turmsiebung:

Angaben in Gew.% (Details zum Messverfahren siehe unter Methoden)

| Probe | <500 µm | 500-600 µm | 600-710 µm | 710-800 µm | 800-900 µm | 900-1000 µm |
|---|---|---|---|---|---|---|
| Beispiel E | 0,2 | 0,2 | 0,1 | 0,5 | 1,6 | 4,2 |
| Beispiel F | 0,4 | 0,0 | 0,2 | 0,5 | 1,5 | 4,3 |

| Probe | 1000-1120 µm | 1120-1250 µm | 1250-1400 µm | 1400-1600 µm | 1600-1700 µm | 1700-2000 µm | > 2000 µm |
|---|---|---|---|---|---|---|---|
| Beispiel E | 9,7 | 12,1 | 13,5 | 25,2 | 13,1 | 19,4 | 0,2 |
| Beispiel F | 11,2 | 14,0 | 15,7 | 26,1 | 10,8 | 15,3 | 0,0 |

### 3. Vergleiche 1 und 2: handelsübliches kristallines Glycin

Es werden 2 Chargen eines handelsüblichen Glycins zu Vergleichszwecken (als Stand der Technik) eingesetzt
Vergleich 1: Glycin krist. geeignet zur Verwendung als Excipient EMPROVE® exp Ph.Eur., BP, JP, USP Art.Nr. 1.00590.9025; Charge: VP708290
Vergleich 2: Glycin krist. geeignet zur Verwendung als Excipient EMPROVE® exp Ph.Eur., BP, JP, USP Art.Nr. 1.00590.9025; Charge: VP709890

### Schüttdichte. Stampfdichte, Hausner-Faktor, Kompressibilitätsindex:

(Details zu den Messverfahren siehe unter Methoden)

| Probe | Schüttdichte (g/ml) | Stampfdichte (g/ml) | Hausner-Faktor | Kompressibilitätsindex (%) | Trocknungsverlust (%) |
|---|---|---|---|---|---|
| Vergleich 1 | 1,01 | 1,15 | 1,14 | 12,5 | 0,02 |
| Vergleich 2 | 1,03 | 1,26 | 1,23 | 18,4 | 0,02 |

Obwohl beide Vergleiche einen sehr niedrigen Wassergehalt und auch vergleichbare Schütt-und Stampfdichten zeigen unterscheiden sie sich in der Höhe des Hausner-Faktors und im Kompressibilitätsindex (gem. Ph Eur 6. Ausgabe, Tabelle 2.9.36-2 "Einteilung des Fließverhaltens" ist Vergleich 1 als "gut" Vergleich 2 als "zufriedenstellend" einzustufen) - in der visuellen Beurteilung zeigt Vergleich 2 im Gegensatz zu Vergleich 1 bereits leichte Verbackungstendenzen wobei sich die Agglomerate jedoch durch leichte Druckanwendung zerstören lassen.

### Partikelverteilung bestimmt über Turmsiebung:

Angaben in Gew.% (Details zum Messverfahren siehe unter Methoden)

| Probe | <32 µm | 32-50 µm | 50-100 µm | 100-150 µm | 150-200 µm | 200-250 µm | 250-300 µm | 300-355 µm |
|---|---|---|---|---|---|---|---|---|
| Vergleich 1 | 0,0 | 0,5 | 1,5 | 5,3 | 6,0 | 8,6 | 12,9 | 8,5 |
| Vergleich 2 | 0,0 | 5,6 | 6,7 | 8,5 | 9,3 | 12,3 | 15,8 | 8,2 |

| Probe | 355-400 µm | 1400-500 µm | 500-600 µm | 600-710 µm | 710-1000 µm | > 1000 µm |
|---|---|---|---|---|---|---|
| Vergleich 1 | 8,2 | 17,0 | 15,9 | 8,6 | 7,0 | 0,0 |
| Vergleich 2 | 8,9 | 13,3 | 7,7 | 2,6 | 1,1 | 0,0 |

### C) Ergebnisse

### Zusammenfassung der Versuchsergebnisse

1. Glycin (erfindungsgemäße Beispiele und Vergleiche) zeigen in der DVS ein identisches Verhalten in der Massenänderung; erst ab >90% r.F. ist überhaupt eine Zunahme der Masse zu erkennen d.h. das Material - egal ob erfindungsgemäße Beispiele oder Vergleiche - ist bis 90% r.F. praktisch nicht hygroskopisch. Die Gewichtszunahmen bei 98 % r.F. zeigen für die schnell verbackenden Vergleiche 1 und 2 sogar eine tendenziell geringere Wasseraufnahme im Vergleich zu den stabilen Beispielen A-F.
2. Veränderungen der Trocknungsverluste nach Stressbelastung sind nicht erkennbar - alle Muster (erfindungsgemäße Beispiele und Vergleiche) verhalten sich gleich und zeigen keine Hygroskopizität
3. Trotz dieser fehlenden Tendenz zur Wasseraufnahme zeigen die Muster bei Lagerung unter Stressbelastung überraschenderweise deutliche Unterschiede in ihrem Fließ(Riesel)verhalten: während die Beispiele A bis F rieselfähig bleiben verbacken die Vergleiche 1 und 2 schon nach kürzester Zeit und sind nicht mehr frei fließend aus den Lagergefäßen zu entnehmen - dies trifft selbst auf den Vergleich 1 mit seinen bzgl. des Fließverhalten sehr guten Ausgangswert zu.
4. Versuche und Vergleiche zeigen dass das kompaktierte Glycin mit seiner besonderen Partikelgröße >700 µm auch nach Lagerung unter Stressbedingungen d.h. erhöhter Temperatur und Luftfeuchte deutlich länger rieselfähig bleibt als das Vergleichsmaterial.
5. Das Auflösungsverhalten der Beispiel A bis F ist auch nach Lagerung praktisch unverändert und in seiner Geschwindigkeit ausreichend für die problemlose Weiterverarbeitung

### Versuchsergebnisse im Detail

### 1) Hygroskopizität (DVS)

Sowohl die erfindungsgemäßen Beispiele A bis F und die Vergleiche 1 und 2 zeigen erst ab einer rel. Feuchte von >90% eine Gewichtszunahme (DVS). Bei einer rel. Feuchte von 98% zeigen die kompaktierten und nicht verklumpenden Beispiele A bis E sogar tendenziell eine leicht stärkere Gewichtszunahme als die Vergleiche 1 und 2.

**DVS-Messung (Dynamic Vapor Sorption):**

| | Relative Feuchte (%) | | | | | | | | | | |
|---|---|---|---|---|---|---|---|---|---|---|---|
| | 0,0 | 10,0 | 20,0 | 30,0 | 40,0 | 50,0 | 60,0 | 70,0 | 80,0 | 90,0 | 98,0 |
| Beispiel A | 0,00 | 0,00 | 0,00 | 0,00 | 0,00 | 0,00 | 0,01 | 0,01 | 0,01 | 0,03 | 2,29 |
| Beispiel B | 0,00 | 0,00 | 0,00 | 0,00 | 0,01 | 0,01 | 0,01 | 0,01 | 0,02 | 0,03 | 3,25 |
| Beispiel C | 0,00 | 0,00 | 0,00 | 0,00 | 0,00 | 0,01 | 0,01 | 0,01 | 0,02 | 0,04 | 2,70 |
| Beispiel D | 0,00 | 0,00 | 0,01 | 0,01 | 0,01 | 0,01 | 0,01 | 0,02 | 0,02 | 0,03 | 3,15 |
| Beispiel E | 0,00 | 0,00 | 0,00 | 0,00 | 0,00 | 0,00 | 0,01 | 0,01 | 0,01 | 0,02 | 2,49 |
| Beispiel F | 0,00 | 0,00 | 0,00 | 0,00 | 0,00 | 0,00 | 0,01 | 0,01 | 0,01 | 0,02 | 2,86 |
| Vergleich 1 | 0,00 | 0,00 | 0,00 | 0,00 | 0,00 | 0,01 | 0,01 | 0,01 | 0,01 | 0,01 | 1,83 |
| Vergleich 2 | 0,00 | 0,00 | 0,00 | 0,00 | 0,00 | 0,00 | 0,00 | 0,00 | 0,01 | 0,02 | 1,40 |

Angabe der Gewichtszunahmen in "Gew. %"

### 2) Trocknungsverluste nach Lagerung unter Stressbedingungen

Angaben in Gew. %

**Lagerbedingung: 25°C/60% r.F. offene Lagerung**

| Muster | Startwert | nach 1 Woche | nach 2 Wochen | nach 7 Wochen |
|---|---|---|---|---|
| Beispiel A | keine Einlagerung erfolgt | | | |
| Beispiel B | keine Einlagerung erfolgt | | | |
| Beispiel C | keine Einlagerung erfolgt | | | |
| Beispiel D | keine Einlagerung erfolgt | | | |
| Beispiel E | 0,03 | 0,05 | 0,05 | 0,09 |
| Beispiel F | 0,03 | 0,09 | 0,02 | 0,09 |
| Vergleich 1 | 0,02 | 0,07 | 0,01 | |
| Vergleich 2 | 0,02 | 0,05 | 0,04 | 0,02 |

**Lagerbedingung: 25°C/60%r.F. geschlossene Lagerung**

| Muster | Startwert | nach 1 Woche | nach 2 Wochen | nach 7 Wochen |
|---|---|---|---|---|
| Beispiel A | keine Einlagerung erfolgt | | | |
| Beispiel B | keine Einlagerung erfolgt | | | |
| Beispiel C | keine Einlagerung erfolgt | | | |
| Beispiel D | keine Einlagerung erfolgt | | | |
| Beispiel E | 0,03 | 0,06 | 0,03 | 0,08 |
| Beispiel F | 0,03 | 0,08 | 0,06 | 0,10 |
| Vergleich 1 | 0,02 | 0,01 | 0,02 | |
| Vergleich 2 | 0,02 | 0,05 | 0,03 | 0,02 |

**Lagerbedingung: 40°C/75%r.F. offene Lagerung**

| Muster | Startwert | nach 1 Woche | nach 2 Wochen | nach 7 Wochen |
|---|---|---|---|---|
| Beispiel A | 0,02 | - | 0,01 | 0,02 |
| Beispiel B | 0,04 | - | 0,04 | < 0,01 |
| Beispiel C | 0,05 | - | < 0,01 | 0,01 |
| Beispiel D | 0,04 | - | < 0,01 | < 0,01 |
| Beispiel E | 0,03 | 0,06 | 0,03 | 0,08 |
| Beispiel F | 0,03 | 0,04 | 0,04 | 0,07 |
| Vergleich 1 | 0,02 | 0,01 | 0,01 | 0,04 |
| Vergleich 2 | 0,02 | 0,04 | 0,03 | 0,03 |

**Lagerbedingung: 40°C/75%r.F. geschlossene Lagerung**

| Muster | Startwert | nach 1 Woche | nach 2 Wochen | nach 7 Wochen |
|---|---|---|---|---|
| Beispiel A | 0,02 | - | < 0,01 | < 0,01 |
| Beispiel B | 0,04 | - | 0,04 | < 0,01 |
| Beispiel C | 0,05 | - | < 0,01 | 0,01 |
| Beispiel D | 0,04 | - | 0,02 | 0,025 |
| Beispiel E | 0,03 | 0,01 | 0,03 | 0,08 |
| Beispiel F | 0,03 | 0,08 | 0,04 | 0,09 |
| Vergleich 1 | 0,02 | 0,03 | 0,02 | 0,04 |
| Vergleich 2 | 0,02 | 0,05 | 0,02 | 0,03 |

### 3) Gewichtsänderungen nach Lagerung unter Stressbedingungen

**Lagerbedingung: 25°C/60% r.F. offene Lagerung**

| Muster | Differenz [g] nach 1 Woche | Differenz [g] nach 2 Wochen | Differenz [g] nach 7 Wochen |
|---|---|---|---|
| Beispiel A | keine Einlagerung erfolgt | | |
| Beispiel B | keine Einlagerung erfolgt | | |
| Beispiel C | keine Einlagerung erfolgt | | |
| Beispiel D | keine Einlagerung erfolgt | | |
| Beispiel E | 0,01 | -0,03 | 0,00 |
| Beispiel F | -0,02 | 0,03 | 0,00 |
| Vergleich 1 | 0,00 | -0,01 | 0,04 |
| Vergleich 2 | 0,01 | 0,07 | 0,08 |

**Lagerbedingung: 25°C/60%r.F. geschlossene Lagerung**

| Muster | Differenz [g] nach 1 Woche | Differenz [g] nach 2 Wochen | Differenz [g] nach 7 Wochen |
|---|---|---|---|
| Beispiel A | keine Einlagerung erfolgt | | |
| Beispiel B | keine Einlagerung erfolgt | | |
| Beispiel C | keine Einlagerung erfolgt | | |
| Beispiel D | keine Einlagerung erfolgt | | |
| Beispiel E | -0,01 | -0,02 | 0,00 |
| Beispiel F | -0,01 | -0,01 | 0,01 |
| Vergleich 1 | 0,00 | -0,01 | -0,02 |
| Vergleich 2 | 0,04 | 0,08 | 0,00 |

**Lagerbedingung: 40°C/75%r.F. offene Lagerung**

| Muster | Differenz [g] nach 1 Woche | Differenz [g] nach 2 Wochen | Differenz [g] nach 7 Wochen |
|---|---|---|---|
| Beispiel A | - | 0,01 | 0,00 |
| Beispiel B | - | 0,00 | -0,01 |
| Beispiel C | - | -0,01 | -0,01 |
| Beispiel D | - | 0,00 | -0,02 |
| Beispiel E | 0,04 | 0,00 | 0,04 |
| Beispiel F | -0,01 | 0,03 | -0,02 |
| Vergleich 1 | -0,02 | 0,03 | 0,00 |
| Vergleich 2 | 0,02 | 0,02 | -0,04 |

**Lagerbedingung: 40°C/75%r.F. geschlossene Lagerung**

| Muster | Differenz [g] nach 1 Woche | Differenz [g] nach 2 Wochen | Differenz [g] nach 7 Wochen |
|---|---|---|---|
| Beispiel A | - | -0,02 | -0,03 |
| Beispiel B | - | -0,02 | -0,03 |
| Beispiel C | - | 0,01 | -0,04 |
| Beispiel D | - | 0,00 | -0,04 |
| Beispiel E | 0,07 | -0,02 | 0,00 |
| Beispiel F | -0,03 | -0,01 | -0,02 |
| Vergleich 1 | 0,03 | 0,01 | -0,06 |
| Vergleich 2 | 0,01 | 0,03 | -0,01 |

### 4) Veränderungen der Auflösungsgeschwindigkeiten nach Lagerung unter Stressbedingungen (Angaben in "Sekunden")

**Lagerbedingung: 25°C/60% r.F. offene Lagerung**

| Muster | Startwert / Tag der Einlagerung | nach 1 Woche | nach 2 Wochen | nach 7 Wochen |
|---|---|---|---|---|
| Beispiel E | 318 ± 20 | 331 ± 13 | 343 ± 7 | 328 ± 19 |
| Beispiel F | 316 ± 15 | 300 ± 7 | 341 ± 10 | 343 ± 20 |
| Vergleich 1 | 233 ±51 | 176 ± 14 | 194 ± 7 | 198 ± 6 |
| Vergleich 2 | 127 ± 11 | 181 ± 8 | 185 ± 5 | 191 ± 9 |

**Lagerbedingung: 25°C/60%r.F. geschlossene Lagerung**

| Muster | Startwert / Tag der Einlagerung | nach 1 Woche | nach 2 Wochen | nach 7 Wochen ( |
|---|---|---|---|---|
| Beispiel E | 318 ± 20 | 328 ± 7 | 352 ± 8 | 358 ± 19 |
| Beispiel F | 316 ± 15 | 301 ± 13 | 339 ± 6 | 363 ± 25 |
| Vergleich 1 | 233 ±51 | 176 ± 13 | 216 ± 6 | 207 ± 20 |
| Vergleich 2 | 127 ± 11 | 173 ± 6 | 209 ±5 | 196 ± 5 |

**Lagerbedingung: 40°C/75%r.F. offene Lagerung**

| Muster | Startwert / Tag der Einlagerung | nach 1 Woche | nach 2 Wochen | nach 7 Wochen |
|---|---|---|---|---|
| Beispiel A | 358 ± 12 | - | 335 ± 11 | 313 ± 5 |
| Beispiel B | 347 ± 11 | - | 340 ± 16 | 309 ± 12 |
| Beispiel C | 339 ± 6 | - | 346 ± 18 | 324 ± 20 |
| Beispiel D | 348 ± 5 | - | 332 ± 9 | 270 ± 10 |
| Beispiel E | 318 ± 20 | 336 ± 12 | 326 ± 22 | 337 ± 10 |
| Beispiel F | 316 ± 15 | 312 ± 18 | 332 ± 13 | 330 ± 12 |
| Vergleich 1 | 233 ±51 | 192 ± 9 | 202 ± 13 | 191 ± 17 |
| Vergleich 2 | 127 ± 11 | 182 ± 5 | 187 ±6 | 209 ± 12 |

**Lagerbedingung: 40°C/75%r.F. geschlossene Lagerung**

| Muster | Startwert / Tag der Einlagerung | nach 1 Woche | nach 2 Wochen | nach 7 Wochen |
|---|---|---|---|---|
| Beispiel A | 358 ± 12 | - | 350 ± 23 | 297 ± 4 |
| Beispiel B | 347 ± 11 | - | 332 ± 12 | 304 ± 9 |
| Beispiel C | 339 ± 6 | - | 326 ± 10 | 299 ± 6 |
| Beispiel D | 348 ± 5 | - | 319 ± 11 | 290 ± 1 |
| Beispiel E | 318 ± 20 | 322 ± 8 | 343 ± 5 | 343 ± 14 |
| Beispiel F | 316 ± 15 | 316 ± 7 | 350 ± 15 | 342 ± 12 |
| Vergleich 1 | 233 ±51 | 177 ± 6 | 215 ± 8 | 203 ± 4 |
| Vergleich 2 | 127 ± 11 | 201 ± 17 | 193 ± 10 | 215 ± 9 |

### 5) Verklumpungsverhalten nach Lagerung unter Stressbedingungen (visuelle Beschreibung)

"Frei fließend" = das Glycin ist ohne Krafteintrag frei rieselnd - Agglomerate sind visuell nicht zu erkennen.

"Klumpig" = das Glycin zeigt vereinzelte größere

Agglomerate/Verkrustungen (von ca. 1 cm Durchmesser) ist jedoch noch frei fließend.

"Leicht verbacken" = das Glycin ist fest; allerdings kann es durch leichten Krafteintrag (leichtes Stochern mit einem Glasstab oder Spatel, Schlagen oder Schütteln) wieder in den frei fließenden Zustand gebracht werden. "Stark verbacken" = das Glycin ist fest; es ist ein starker Krafteintrag (starkes Stochern mit einem Glasstab oder Spatel) notwendig, um das Glycin wieder aufzulockern - allerdings ist das Glycin danach immer noch stark verklebt (Brockenbildung) und nicht frei fließend.

**Lagerbedingung: 25°C/60% r.F. offene Lagerung**

| Muster | Startwert | nach 1 Woche | nach 2 Wochen | nach 7 Wochen |
|---|---|---|---|---|
| Beispiel E | frei fließend | frei fließend | frei fließend | frei fließend |
| Beispiel F | frei fließend | frei fließend | frei fließend | frei fließend |
| Vergleich 1 | frei fließend | leicht verbacken | leicht verbacken | leicht verbacken |
| Vergleich 2 | klumpig | stark verbacken | stark verbacken | stark verbacken |

**Lagerbedingung: 25°C/60%r.F. geschlossene Lagerung**

| Muster | Startwert | nach 1 Woche | nach 2 Wochen | nach 7 Wochen |
|---|---|---|---|---|
| Beispiel E | frei fließend | frei fließend | frei fließend | frei fließend |
| Beispiel F | frei fließend | frei fließend | frei fließend | frei fließend |
| Vergleich 1 | frei fließend | klumpig | frei fließend | leicht verbacken |
| Vergleich 2 | klumpig | stark verbacken | stark verbacken | stark verbacken |

**Lagerbedingung: 40°C/75%r.F. offene Lagerung**

| Muster | Startwert | nach 1 Woche | nach 2 Wochen | nach 7 Wochen |
|---|---|---|---|---|
| Beispiel A | frei fließend | - | leicht verbacken | leicht verbacken |
| Beispiel B | frei fließend | - | leicht verbacken | leicht verbacken |
| Beispiel C | frei fließend | - | leicht verbacken | leicht verbacken |
| Beispiel D | frei fließend | - | leicht verbacken | leicht verbacken |
| Beispiel E | frei fließend | leicht verbacken | leicht verbacken | leicht verbacken |
| Beispiel F | frei fließend | leicht verbacken | leicht verbacken | leicht verbacken |
| Vergleich 1 | frei fließend | stark verbacken | stark verbacken | stark verbacken |
| Vergleich 2 | klumpig | stark verbacken | stark verbacken | stark verbacken |

**Lagerbedingung: 40°C/75%r.F. geschlossene Lagerung**

| Muster | Startwert | nach 1 Woche | nach 2 Wochen | nach 7 Wochen |
|---|---|---|---|---|
| Beispiel A | frei fließend | - | frei fließend | frei fließend (schwache Agglomerate) |
| Beispiel B | frei fließend | - | frei fließend | frei fließend |
| Beispiel C | frei fließend | - | frei fließend | frei fließend |
| Beispiel D | frei fließend | - | frei fließend | frei fließend |
| Beispiel E | frei fließend | frei fließend | frei fließend | frei fließend |
| Beispiel F | frei fließend | frei fließend | frei fließend | frei fließend |
| Vergleich 1 | frei fließend | stark verbacken | stark verbacken | stark verbacken |
| Vergleich 2 | klumpig | stark verbacken | stark verbacken | stark verbacken |

### 6) Veränderungen des Fließverhaltens (gemessen als Änderung des "Avalanche Angle" im Revolution Powder Analyzer) nach Lagerung unter Stressbedingungen

**Lagerbedingung: 25°C/60% r.F. offene Lagerung**

| | Avalanche Angle [°] | | | |
|---|---|---|---|---|
| Muster | Startwert | nach 1 Woche | nach 2 Wochen | nach 7 Wochen |
| Beispiel E | 43,6 | 42,5 | 41,8 | 42,1 |
| Beispiel F | 45,6 | 43,2 | 41,9 | 42,2 |
| Vergleich 1 | 39,4 | 40,8 | 39,5 | 40,2 |
| Vergleich 2 | 65,6 | n.b. | n.b. | n.b. |

**Lagerbedingung: 25°C/60%r.F. geschlossene Lagerung**

| | Avalanche Angle [°] | | | |
|---|---|---|---|---|
| Muster | Startwert | nach 1 Woche | nach 2 Wochen | nach 7 Wochen |
| Beispiel E | 43,6 | 42,6 | 41,8 | 42,1 |
| Beispiel F | 45,6 | 42,8 | 43,2 | 42,3 |
| Vergleich 1 | 39,4 | 41,8 | 39,6 | 40,5 |
| Vergleich 2 | 65,6 | n.b. | n.b. | n.b. |

**Lagerbedingung: 40°C/75%r.F. offene Lagerung**

| | Avalanche Angle [°] | | | |
|---|---|---|---|---|
| Muster | Startwert | nach 1 Woche | nach 2 Wochen | nach 7 Wochen |
| Beispiel E | 43,6 | 43,8 | 43,2 | 43,5 |
| Beispiel F | 45,6 | 43,7 | 42,7 | 42,6 |
| Vergleich 1 | 39,4 | n.b. | n.b. | n.b. |
| Vergleich 2 | 65,6 | n.b. | n.b. | n.b. |

**Lagerbedingung: 40°C/75%r.F. geschlossene Lagerung**

| | Avalanche Angle [°] | | | |
|---|---|---|---|---|
| Muster | Startwert | nach 1 Woche | nach 2 Wochen | nach 7 Wochen |
| Beispiel E | 43,6 | 42,8 | 42,8 | 42,9 |
| Beispiel F | 45,6 | 43,4 | 42,4 | 42,8 |
| Vergleich 1 | 39,4 | n.b. | n.b. | n.b. |
| Vergleich 2 | 65,6 | n.b. | n.b. | n.b. |

n.b. = nicht bestimmbar da das Material schon zu fest verbacken ist um es aus dem Gefäß zu entnehmen (es sind zu viele Agglomerate vorhanden bzw. das Material muss aus der Schale/Flasche herausgestochert werden)

### 7) Veränderungen des Fließverhaltens (gemessen als Änderung der "Break Energy" im Revolution Powder Analyzer) nach Lagerung unter Stressbedingungen

**Lagerbedingung: 25°C/60% r.F. offene Lagerung**

| | Break Energy [mJ] | | | |
|---|---|---|---|---|
| Muster | Startwert | nach 1 Woche | nach 2 Wochen | nach 7 Wochen |
| Beispiel E | 28,4 | 28,1 | 27,7 | 27,9 |
| Beispiel F | 28,8 | 28,6 | 28,3 | 28,2 |
| Vergleich 1 | 27,4 | 27,5 | 27,2 | 27,5 |
| Vergleich 2 | 33,5 | n.b. | n.b. | n.b. |

**Lagerbedingung: 25°C/60%r.F. geschlossene Lagerung**

| | Break Energy [mJ] | | | |
|---|---|---|---|---|
| Muster | Startwert | nach 1 Woche | nach 2 Wochen | nach 7 Wochen |
| Beispiel E | 28,4 | 28,1 | 27,7 | 27,9 |
| Beispiel F | 28,8 | 28,5 | 28,6 | 27,9 |
| Vergleich 1 | 27,4 | 28,0 | 27,1 | 27,4 |
| Vergleich 2 | 33,5 | n.b. | n.b. | n.b. |

**Lagerbedingung: 40°C/75%r.F. offene Lagerung**

| | Break Energy [mJ] | | | |
|---|---|---|---|---|
| Muster | Startwert | nach 1 Woche | nach 2 Wochen | nach 7 Wochen |
| Beispiel E | 28,4 | 28,7 | 28,0 | 28,0 |
| Beispiel F | 28,8 | 28,7 | 28,3 | 28,1 |
| Vergleich 1 | 27,4 | n.b. | n.b. | n.b. |
| Vergleich 2 | 33,5 | n.b. | n.b. | n.b. |

**Lagerbedingung: 40°C/75%r.F. geschlossene Lagerung**

| | Break Energy [mJ] | | | |
|---|---|---|---|---|
| Muster | Startwert | nach 1 Woche | nach 2 Wochen | nach 7 Wochen |
| Beispiel E | 28,4 | 28,3 | 28,4 | 28,2 |
| Beispiel F | 28,8 | 28,9 | 28,0 | 28,5 |
| Vergleich 1 | 27,4 | n.b. | n.b. | n.b. |
| Vergleich 2 | 33,5 | n.b. | n.b. | n.b. |

n.b. = nicht bestimmbar da das Material schon zu fest verbacken ist um es aus dem Gefäß zu entnehmen (es sind zu viele Agglomerate vorhanden bzw. das Material muss aus der Schale/Flasche herausgestochert werden)

## Patentansprüche

1. Glycin-Granulat, wobei mindestens 75% (w/w) des Glycin-Granulats eine Korngröße von mindestens 0,7 mm aufweist.

2. Glycin-Granulat nach Anspruch 1, **dadurch gekennzeichnet, dass** mindestens 80% (w/w) des Glycin-Granulats eine Korngröße von mindestens 0,8 mm aufweist.

3. Glycin-Granulat nach Anspruch 1 oder 2, **dadurch gekennzeichnet, dass** das Glycin-Granulat eine Schüttdichte kleiner oder gleich 0,9 g/ml aufweist.

4. Glycin-Granulat nach einem oder mehreren der Ansprüche 1 bis 3, **dadurch gekennzeichnet, dass** das Glycin-Granulat nach Lagerung über 3 Monate in einem verschlossenen Gebinde bei Raumtemperatur rieselfähig ist.

5. Glycin-Granulat nach Anspruch 1, herstellbar durch Kompaktierung.

6. Glycin-Granulat nach Anspruch 5, **dadurch gekennzeichnet, dass** die Herstellung erfolgt durch
a) Bereitstellen von Glycin-Pulver
b) Kompaktieren des Glycin-Pulvers aus Schritt a) in einem Rollenkompaktor.

7. Glycin-Granulat nach Anspruch 5 oder 6, **dadurch gekennzeichnet, dass** in Schritt b) die Presskraft des Rollenkompaktors zwischen 1 und 50 KN/cm Rollenweite liegt.

8. Glycin nach einem oder mehreren der Ansprüche 5 bis 7, **dadurch gekennzeichnet, dass** die Herstellung erfolgt durch
a)Bereitstellen von Glycin-Pulver
b)Kompaktieren des Glycin-Pulvers aus Schritt a) in einem Rollenkompaktor, wobei ein Kompaktat entsteht, das Partikel mit einer Korngröße über 0,7 mm enthält
c)Zumindest teilweise Rückführung von Glycin-Partikeln aus dem in Schritt b) erhaltenen Kompaktat, die eine Korngröße kleiner 0,7 mm haben, zu dem in Schritt a) bereitgestellten Glycin-Pulver.

9. Glycin nach einem oder mehreren der Ansprüche 5 bis 8, **dadurch gekennzeichnet, dass** die Rückführung in Schritt c) erfolgt durch Brechung der Kompaktate und Klassierung nach Korngröße, wobei Glycin-Partikeln, die eine Korngröße kleiner 0,7 mm aufweisen, zumindest teilweise zu dem in Schritt a) bereitgestellten Glycin-Pulver rückgeführt werden.

10. Verfahren zur Herstellung von Glycin-Granulat, wobei mindestens 75% (w/w) des Glycin-Granulats eine Korngröße von mindestens 0,7 mm aufweist, durch
a) Bereitstellen von Glycin-Pulver
b) Kompaktieren des Glycin-Pulvers aus Schritt a).

11. Verfahren nach Anspruch 10, **dadurch gekennzeichnet, dass** die Herstellung erfolgt durch
a) Bereitstellen von Glycin-Pulver
b) Kompaktieren des Glycin-Pulvers aus Schritt a) in einem Rollenkompaktor, wobei ein Kompaktat entsteht, das Partikel mit einer Korngröße über 0,7 mm enthält
c) Zumindest teilweise Rückführung von Glycin-Partikeln aus dem in Schritt b) erhaltenen Kompaktat, die eine Korngröße kleiner 0,7 mm haben, zu dem in Schritt a) bereitgestellten Glycin-Pulver.

12. Verfahren nach Anspruch 10 oder 11, **dadurch gekennzeichnet, dass** in Schritt b) die Presskraft des Rollenkompaktors zwischen 1 und 50 KN/cm Rollenweite liegt.

13. Verfahren nach einem oder mehreren der Ansprüche 10 bis 12, **dadurch gekennzeichnet, dass** die Rückführung in Schritt c) erfolgt durch Brechung der Kompaktate und Klassierung nach Korngröße, wobei Glycin-Partikeln, die eine Korngröße kleiner 0,7 mm haben, zumindest teilweise zu dem in Schritt a) bereitgestellten Glycin-Pulver rückgeführt werden.

14. Verwendung von Glycin-Granulat, wobei mindestens 75% (w/w) des Glycin-Granulats eine Korngröße von mindestens 0,7 mm aufweist, zur Herstellung von Nährmedien für medizinische Anwendungen oder in der Biotechnologie.

15. Verwendung nach Anspruch 14, **dadurch gekennzeichnet, dass** das Glycin-Granulat kompaktiert ist.

## Claims

1. Glycine granules, where at least 75% (w/w) of the glycine granules have a particle size of at least 0.7 mm

2. Glycine granules according to Claim 1, **characterised in that** at least 80% (w/w) of the glycine granules have a particle size of at least 0.8 mm.

3. Glycine granules according to Claim 1 or 2, **characterised in that** the glycine granules have a bulk density of less than or equal to 0.9 g/ml.

4. Glycine granules according to one or more of Claims 1 to 3, **characterised in that** the glycine granules are flowable after storage for 3 months in a closed container at room temperature.

5. Glycine granules according to Claim 1, which can be prepared by compaction.

6. Glycine granules according to Claim 5, **characterised in that** the preparation is carried out by
a) provision of glycine powder
b) compaction of the glycine powder from step a) in a roller compactor.

7. Glycine granules according to Claim 5 or 6, **characterised in that** the pressing force of the roller compactor in step b) is between 1 and 50 kN/cm of roller width.

8. Glycine granules according to one or more of Claims 5 to 7, **characterised in that** the preparation is carried out by
a) provision of glycine powder
b) compaction of the glycine powder from step a) in a roller compactor, giving a compact which comprises particles having a particle size above 0.7 mm
c) at least partial recycling of glycine particles from the compact obtained in step b) that have a particle size smaller than 0.7 mm to the glycine powder provided in step a).

9. Glycine granules according to one or more of Claims 5 to 8, **characterised in that** the recycling in step c) is carried out by crushing the compacts and classification by particle size, where at least some of the glycine particles that have a particle size smaller than 0.7 mm are recycled to the glycine powder provided in step a).

10. Process for the preparation of glycine granules, where at least 75% (w/w) of the glycine granules have a particle size of at least 0.7 mm, by
a) provision of glycine powder
b) compaction of the glycine powder from step a).

11. Process according to Claim 10, **characterised in that** the preparation is carried out by
a) provision of glycine powder
b) compaction of the glycine powder from step a) in a roller compactor, giving a compact which comprises particles having a particle size above 0.7 mm
c) at least partial recycling of glycine particles from the compact obtained in step b) that have a particle size smaller than 0.7 mm to the glycine powder provided in step a).

12. Process according to Claim 10 or 11, **characterised in that** the pressing force of the roller compactor in step b) is between 1 and 50 kN/cm of roller width.

13. Process according to one or more of Claims 10 to 12, **characterised in that** the recycling in step c) is carried out by crushing the compacts and classification by particle size, where at least some of the glycine particles that have a particle size smaller than 0.7 mm are recycled to the glycine powder provided in step a).

14. Use of glycine granules, where at least 75% (w/w) of the glycine granules have a particle size of at least 0.7 mm, for the preparation of nutrient media for medical applications or in biotechnology.

15. Use according to Claim 14, **characterised in that** the glycine granules have been compacted.

## Revendications

1. Granules de glycine, dans lesquels au moins 75 % (poids/poids) des granules de glycine présentent une taille de particule d'au moins 0,7 mm.

2. Granules de glycine selon la revendication 1, **caractérisés en ce qu'**au moins 80 % (poids/poids) des granules de glycine présentent une taille de particule d'au moins 0,8 mm.

3. Granules de glycine selon la revendication 1 ou 2, **caractérisés en ce que** les granules de glycine présentent une masse volumique apparente inférieure ou égale à 0,9 g/ml.

4. Granules de glycine selon une ou plusieurs des revendications 1 à 3, **caractérisés en ce que** les granules de glycine sont fluables après stockage pendant 3 mois dans un conteneur fermé à température ambiante.

5. Granules de glycine selon la revendication 1, lesquels peuvent être préparés par compactage.

6. Granules de glycine selon la revendication 5, **caractérisés en ce que** la préparation est mise en œuvre au moyen des actions qui suivent :
a) la fourniture de poudre de glycine ; et
b) le compactage de la poudre de glycine de l'étape a) dans un compacteur à rouleau.

7. Granules de glycine selon la revendication 5 ou 6, **caractérisés en ce que** la force de pression du compacteur à rouleau au niveau de l'étape b) est entre 1 et 50 kN/cm de largeur de rouleau.

8. Granules de glycine selon une ou plusieurs des revendications 5 à 7, **caractérisés en ce que** la préparation est mise en œuvre au moyen des actions qui suivent :
a) la fourniture de poudre de glycine ;
b) le compactage de la poudre de glycine de l'étape a) dans un compacteur à rouleau, d'où l'obtention d'une matière compacte qui comprend des particules qui présentent une taille de particule au-delà de 0,7 mm ; et
c) un recyclage au moins partiel de particules de glycine provenant de la matière compacte qui est obtenue au niveau de l'étape b) et qui présentent une taille de particule inférieure à 0,7 mm selon la poudre de glycine qui est fournie au niveau de l'étape a).

9. Granules de glycine selon une ou plusieurs des revendications 5 à 8, **caractérisés en ce que** le recyclage de l'étape c) est mis en œuvre en écrasant les matières compactes et par classification par taille de particule, où au moins certaines des particules de glycine qui présentent une taille de particule inférieure à 0,7 mm sont recyclées selon la poudre de glycine qui est fournie au niveau de l'étape a).

10. Procédé pour la préparation de granules de glycine, dans lequel au moins 75 % (poids/poids) des granules de glycine présentent une taille de particule d'au moins 0,7 mm, au moyen des actions qui suivent :
a) la fourniture de poudre de glycine ; et
b) le compactage de la poudre de glycine de l'étape a).

11. Procédé selon la revendication 10, **caractérisé en ce que** la préparation est mise en œuvre au moyen des étapes qui suivent :
a) la fourniture de poudre de glycine ;
b) le compactage de la poudre de glycine de l'étape a) dans un compacteur à rouleau, d'où l'obtention d'une matière compacte qui comprend des particules qui présentent une taille de particule au-delà de 0,7 mm ; et
c) un recyclage au moins partiel de particules de glycine provenant de la matière compacte qui est obtenue au niveau de l'étape b) et qui présentent une taille de particule inférieure à 0,7 mm selon la poudre de glycine qui est fournie au niveau de l'étape a).

12. Procédé selon la revendication 10 ou 11, **caractérisé en ce que** la force de pression du compacteur à rouleau au niveau de l'étape b) est entre 1 et 50 kN/cm de largeur de rouleau.

13. Procédé selon une ou plusieurs des revendications 10 à 12, **caractérisé en ce que** le recyclage de l'étape c) est mis en œuvre en écrasant les matières compactes et par classification par taille de particule, où au moins certaines des particules de glycine qui présentent une taille de particule inférieure à 0,7 mm sont recyclées selon la poudre de glycine qui est fournie au niveau de l'étape a).

14. Utilisation de granules de glycine, dans laquelle au moins 75 % (poids/ poids) des granules de glycine présentent une taille de particule d'au moins 0,7 mm, pour la préparation de milieux nutritifs de culture pour les applications médicales ou en biotechnologie.

15. Utilisation selon la revendication14, **caractérisée en ce que** les granules de glycine ont été compactés.
